(19) Europäisches Patentamt / European Patent Office / Office européen des brevets

(11) **EP 3 594 674 B1**

(12) **EUROPEAN PATENT SPECIFICATION**

(45) Date of publication and mention
of the grant of the patent:
**05.03.2025 Bulletin 2025/10**

(21) Application number: **18764749.0**

(22) Date of filing: **18.01.2018**

(51) International Patent Classification (IPC):
**G01N 27/447** (2006.01)     **C12Q 1/68** (2018.01)

(52) Cooperative Patent Classification (CPC):
(C-Sets available)
**G01N 27/447; C12Q 1/6806**     (Cont.)

(86) International application number:
**PCT/JP2018/001395**

(87) International publication number:
**WO 2018/163610 (13.09.2018 Gazette 2018/37)**

(54) **ELECTROPHORETIC ANALYSIS METHOD, ELECTROPHORETIC ANALYSIS DEVICE, AND ELECTROPHORETIC ANALYSIS PROGRAM**

ELEKTROPHORETISCHES ANALYSEVERFAHREN, ELEKTROPHORETISCHE ANALYSEVORRICHTUNG UND ELEKTROPHORETISCHES ANALYSEPROGRAMM

PROCÉDÉ D'ANALYSE ÉLECTROPHORÉTIQUE, DISPOSITIF D'ANALYSE ÉLECTROPHORÉTIQUE ET PROGRAMME D'ANALYSE ÉLECTROPHORÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.03.2017   JP 2017046209**

(43) Date of publication of application:
**15.01.2020   Bulletin 2020/03**

(73) Proprietor: **Shimadzu Corporation
Kyoto-shi, Kyoto 604-8511 (JP)**

(72) Inventor: **CHINOMI, Kenta
Kyoto-shi
Kyoto 604-8511 (JP)**

(74) Representative: **Kilian Kilian & Partner mbB
Zielstattstraße 23a
81379 München (DE)**

(56) References cited:
**JP-A- 2007 526 979     JP-A- 2012 501 458
JP-A- 2015 519 887**

- AUER H ET AL: "Chipping away at the chip bias: RNA degradation in microarray analysis", NATURE GENETICS, NATURE PUBLISHING GROUP, NEW YORK, US, vol. 35, no. 4, December 2003 (2003-12-01), pages 292 - 293, XP002318708, ISSN: 1061-4036, DOI: 10.1038/NG1203-292
- AUER H ET AL: "Chipping away at the chip bias: RNA degradation in microarray analysis - Supplementary Information: Supplementary Methods", NATURE GENETICS, December 2003 (2003-12-01), pages 1 - 4, XP055741083, Retrieved from the Internet <URL:https://www.nature.com/articles/ng1203-292> [retrieved on 20201016], DOI: 10.1038/ng1203-292
- SCHROEDER ANDREAS ET AL: "The RIN: an RNA integrity number for assigning integrity values to RNA measurements", BMC MOLECULAR BIOLOGY, BIOMED CENTRAL LTD, GB, vol. 7, no. 1, 31 January 2006 (2006-01-31), pages 3, XP021014979, ISSN: 1471-2199, DOI: 10.1186/1471-2199-7-3

- **ANDREAS SCHROEDER ET AL: "The RIN: an RNA integrity number for assigning integrity values to RNA measurements - Additional file 2 (online): Description of the total set of features computed", BMC MOLECULAR BIOLOGY, 31 January 2006 (2006-01-31), pages 1 - 2, XP055741282, Retrieved from the Internet <URL:https://bmcmolbiol.biomedcentral.com/articles/10.1186/1471-2199-7-3> [retrieved on 20201019], DOI: 10.1186/1471-2199-7-3**
- **FLEIGE, SIMONE ET AL.: "RNA integrity and the effect on the real-time qRT-PCR performance", MOLECULAR ASPECTS OF MEDICINE, vol. 27, no. 2-3, April 2006 (2006-04-01), pages 126 - 139, XP055542654**
- **MUELLER, ODILO ET AL.: "RNA Integrity Number (RIN) - Standardization of RNA Quality Control", AGILENT TECHNOLOGIES, 1 May 2004 (2004-05-01), pages 1 - 8, XP055542656, Retrieved from the Internet <URL:http://gene-quantification.net/RIN.pdf> [retrieved on 20180315]**
- **FONTANESI, L. ET AL.: "Evaluation of post mortem stability of porcine skeletal muscle RNA", MEAT SCIENCE, vol. 80, no. 4, 14 June 2008 (2008-06-14), pages 1345 - 1351, XP025465925**

(52) Cooperative Patent Classification (CPC): (Cont.)

C-Sets
**C12Q 1/6806, C12Q 2565/125**

## Description

## Technical Field

The present invention relates to an electrophoretic analysis method, an electrophoretic analysis device, and an electrophoretic analysis program for evaluating quality of an RNA molecule by a waveform analysis of an electrophoresis waveform.

## Background Art

When evaluating quality of RNA (ribonucleic acid), in some cases, an electrophoretic analysis device is used. RNA is decomposed and degraded due to various factors, such as, e.g., enzyme (RNase), heat, and ultraviolet light. The degree of degradation of RNA can be evaluated by analyzing the shape of the electrophoresis waveform (electropherogram) obtained by electrophoresis for an RNA sample. The RNA degradation information obtained by the waveform analysis of the electrophoresis waveform is treated as an important parameter in a gene expression research.

As RNA degradation information, various indexes, such as, e.g., RIN, RINe, RQS, RQI, RQN, and RIS, are provided. These degradation indexes are values calculated using a specific waveform region (time range) in an electrophoresis waveform obtained from an RNA sample, and the waveform region to be used differs for each index.

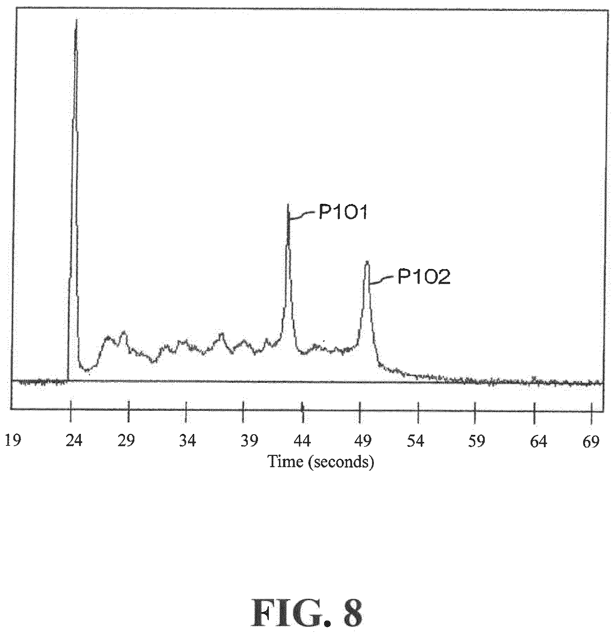

FIG. 8 is a diagram for describing each of the waveform regions in an electrophoresis waveform obtained from an RNA sample. As shown in FIG. 8, the electrophoresis waveform obtained from the RNA sample contains the peak of the 18S fragment (18S peak P101) and the peak of the 28S fragment (28S peak P102). Each degradation index as described above is calculated using the 18S peak P101 and the 28S peak P102.

An RIN, which is one example of a degradation index, is a degradation index used in, for example, 2100 Bio Analyzer provided by Agilent Technologies, Inc. This degradation index RIN is calculated using a waveform region including an 18S peak and a waveform region including a 28S peak, as well as other waveform regions such as a waveform region including a 5S peak (see, for example, Patent Document 1 and Non-Patent Document 1 below).

An RQI, which is another example of a degradation index, is a degradation index used in, for example, Experion provided by Bio-Rad Laboratories, Inc. This degradation index RQI is calculated using a waveform region including an 18S peak and a waveform region including a 28S peak, as well as other waveform regions such as a waveform region immediately before the 18S peak (see, for example, Patent Document 2 and Non-Patent Document 2 below).

An RQS, which is yet another example of a degradation index, is a degradation index used in, for example, LabChip GX provided by PerkinElmer, Inc. In this degradation index RQS, a degradation index is calculated by linear combination of four feature values based on an 18S peak and a 28S peak (see, for example, Non-Patent Document 3 below).

A further electrophoretic analysis method is disclosed in Non-Patent Document 4 below.

## Prior Art Document

## Patent Document

Patent Document 1: Japanese Patent No. 4664280
Patent Document 2: Japanese Patent No. 5620382

## Non-Patent Document

**Non-Patent Document 1:**
Andreas Schroeder, and 9 others, "The RIN: an RNA integrity number for assigning integrity values to RNA measurements", [online], Jan. 31, 2006, BioMed Central, [Feb. 9, 2017 searched], Internet <URL https://www.ncbi.nlm.nih.gov/pmc/articles/PMC1413964/>

**Non-Patent Document** 2:
Vladimir Denisov, and 4 others, "Development and validation of RQI: an RNA quality indicator for the Experion automated electrophoresis system", "online", 2008, Bio-Rad Laboratories, Inc., [February 9, 2017 searched], Internet [URL : http://www.gene-quantification.com/Bio-Rad-bulletin-5761.pdf)

**Non-Patent Document 3:**
"RNA Quality Score (RQS) Calculation and Correlation to RIN", [online], November 9, 2009, Caliper Life Sciences, Inc., [February 9, 2017 search], Internet <URL: https://rtsf.natsci.msu.edu/genomics/tech-notes/caliper-gx-rin-calculations/>

**Non-Patent Document 4:**
Auer H. et al.: "Chipping away at the chip bias: RNA degradation in microarray analysis", Nature Genetics, Nature Publushing Group, New York, US, vol. 35, no. 4, December 2003 (2003-12), pages 292-293, XP002318708, ISSN: 1061-4036, DOI: 10.1038/NG1203-292

## SUMMARY OF THE INVENTION

## Problems to be Solved by the Invention

The above-mentioned conventionally used degradation indexes are calculated using an 18S peak and a 28S peak, but these 18S peak and 28S peak have a feature that the peak intensity decreases with the degradation of RNA and disappears over time. Therefore, there is a problem that when evaluating the quality of RNA that has degraded to a certain extent, it cannot be evaluated accurately.

The present invention has been made in view of the aforementioned circumstances, and an object of the present invention is to provide an electrophoretic analysis method, an electrophoretic analysis device, and an electrophoretic analysis program capable of accurately evaluating quality of RNA even for a degraded RNA.

**Means for Solving the Problems**

The inventor of the present invention has found that, as a result of intensive investigations, a degradation product peak appearing in accordance with degradation of RNA is present in a region (first region) on the low-molecular-weight side of the 18S peak in the RNA electrophoresis waveform, and shifts to the low-molecular-weight side as the RNA degrades. More specifically, the 18S peak and the 28S peak are present in the electrophoresis waveform of RNA before degradation, but these 18S peak and 28S peak decrease in peak intensity as the RNA degrades, and after the 28S peak disappears, the 18S peak disappears. Then, when the RNA is further degraded, a degradation product peak appears, shifts to the low-molecular-weight side as the RNA degrades, and disappears over time.

(1) An electrophoretic analysis method according to the present invention is an electrophoretic analysis method for evaluating quality of RNA by a waveform analysis of an electrophoresis waveform, comprising:
evaluating quality of RNA on a basis of a feature value corresponding to a position of a degradation product peak that appears in accordance with degradation of RNA in a region on a low-molecular-weight side relative to an 18S peak in the electrophoresis waveform.

According to such a configuration, the quality of degraded RNA can be evaluated on the basis of the feature value corresponding to the position of the degradation product peak in the electrophoresis waveform. That is, the degradation product peak appears in a region on the low-molecular-weight side relative to an 18S peak in accordance with the degradation of the RNA after the RNA has degraded to the extent that the 18S peak disappears, and shifts to the low-molecular-weight side as the RNA degrades. Therefore, even for a degraded RNA, the quality can be evaluated with high accuracy on the basis of the feature value corresponding to the position of this degradation product peak.

(2) The feature value corresponding to the position of the degradation product peak may be a value representing a position of a peak top of the degradation product peak.

According to such a configuration, the feature value corresponding to the position of the degradation product peak can be accurately represented using the position of the peak top of the degradation product peak. Therefore, on the basis of the feature value, it is possible to accurately evaluate the quality of RNA even if it is a degraded RNA.

(3) The feature value corresponding to the position of the degradation product peak may be a value representing an area ratio when an area of the degradation product peak is divided into a low-molecular-weight side region and a high-molecular-weight side region.

According to such a configuration, the feature value corresponding to the position of the degradation product peak can be accurately represented using the area ratio when an area of the degradation product peak is divided into the low-molecular-weight side region and the high-molecular-weight side region. Therefore, on the basis of the feature value, it is possible to accurately evaluate the quality of RNA even if it is a degraded RNA.

(4) The feature value corresponding to the position of the degradation product peak may be a value representing a centroid of the degradation product peak.

According to such a configuration, the feature value corresponding to the position of the degradation product peak can be accurately represented using the centroid of the degradation product peak. Therefore, on the basis of the feature value, it is possible to accurately evaluate the quality of RNA even if it is a degraded RNA.

(5) The quality of RNA may be evaluated using the feature value corresponding to the position of the degradation product peak and a feature value based on the 18S peak or a 28S peak.

According to such a configuration, the quality of RNA can be evaluated using the feature value based on the 18S peak or the 28S peak until the RNA has degraded to a certain extent, and the quality of RNA can be evaluated using the feature value corresponding to the position of the degradation product peak after the RNA has degraded to a certain extent. Therefore, the degradation state of RNA can be evaluated over the wider region.

(6) An electrophoretic analysis device according to the present invention is an electrophoretic analysis device for evaluating quality of RNA by a waveform analysis of an electrophoresis waveform, comprising:
a quality value calculation unit configured to calculate a quality value representing quality of RNA on a basis of a feature value corresponding to a position of a degradation product peak that appears in accordance with degradation of RNA in a region on a low-molecular-weight side relative to an 18S peak in the electrophoresis waveform.

(7) An electrophoretic analysis program according to the present invention is an electrophoretic analysis program for evaluating quality of RNA by a waveform analysis of an electrophoresis waveform, the electrophoretic analysis program makes a computer function as a quality value calculation unit for calculating a quality value representing quality of RNA on a basis of a feature value corresponding to a position of a degradation product peak that appears in accordance with degradation of RNA in a region on a low-molecular-weight side relative to an 18S peak in the electrophoresis waveform.

**Effects of the Invention**

According to the present invention, the degradation product peak appears in a region on the low-molecular-weight side relative to the 18S peak in accordance with degradation of RNA after RNA has degraded to the extent that the 18S peak disappears, and shifts to the low-molecular-weight side as the RNA degrades, and therefore, even for a degraded RNA, the quality can be evaluated with high accuracy on the basis of the feature value corresponding to the position of this degradation product peak.

**BRIEF DESCRIPTION OF THE DRAWINGS**

FIG. 1 is a block diagram showing an electrical configuration of an electrophoretic analysis device according to an embodiment of the present invention.

FIG. 2 is a diagram showing electrophoresis waveforms obtained using 12 (twelve) RNA different in quality as samples.

FIG. 3 is a diagram for explaining an area ratio of FPF and FPL.

FIG. 4 is an experimental result showing the relationship between each feature value and quality of RNA corresponding to the position of the degradation product peak.

FIG. 5 is a flowchart showing processing when a data processing unit calculates a quality value.

FIG. 6 is a flowchart showing processing according to a first modification when a data processing unit calculates a quality value.

FIG. 7 is a flowchart showing processing according to a second modification when a data processing unit calculates quality value.

FIG. 8 is a diagram for explaining each of the waveform regions in an electrophoresis waveform obtained from an RNA sample.

**EMBODIMENT FOR CARRYING OUT THE INVENTION**

**1. Electrical Configuration of Electrophoretic Analysis Device**

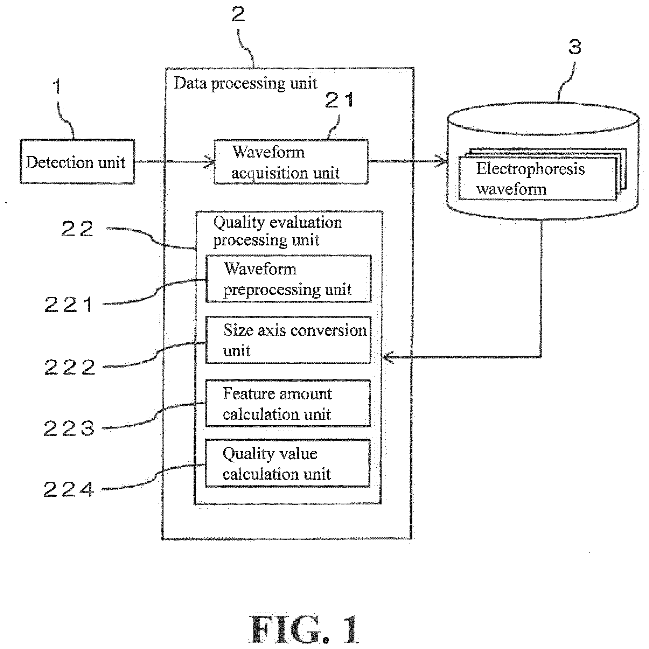

FIG. 1 is a block diagram showing an electrical configuration of an electrophoretic analysis device according to an embodiment of the present invention. This electrophoretic analysis device is a device for separating components in a sample using electrophoresis and detecting the separated components with a detection unit 1. The electrophoresis analysis device according to this embodiment is provided with, for example, a microchip (not shown) in which a flow passage for a sample is formed, and is configured to be able to electrophorese a liquid sample by injecting the liquid sample into the flow passage filled with an electrophoresis medium (separation buffer) and applying a predetermined voltage.

The electrophoretic analysis device is provided with a data processing unit 2 and a storage unit 3 in addition to the detection unit 1 described above. The data processing unit 2 is configured to include, for example, a CPU (Central Processing Unit), and functions as a waveform acquisition unit 21 and a quality evaluation processing unit 22 by the CPU executing a program. The storage unit 3 is configured by, for example, a ROM (Read-Only Memory), a RAM (Random-Access Memory), and a hard disk.

The waveform acquisition unit 21 acquires data of an electrophoresis waveform based on the detection signal from the detection unit 1 and stores the data in the storage unit 3. The electrophoresis waveform is waveform data in which the intensity of the detection signal in the detection unit 1 is associated with the elapsed time, and a peak corresponding to each component in the sample separated by electrophoresis appears.

The quality evaluation processing unit 22 performs processing of evaluating quality of a sample by a waveform analysis of an electrophoresis waveform stored in the storage unit 3. In this embodiment, RNA (ribonucleic acid) is used as a sample, and the case of evaluating quality of RNA by electrophoresis will be described. The quality evaluation processing unit 22 includes, for example, a waveform preprocessing unit 221, a size axis conversion unit 222, a feature amount calculation unit 223, and a quality value calculation unit 224.

The waveform preprocessing unit 221 executes various kinds of preprocessing, such as, e.g., noise removal and baseline correction, on the electrophoresis waveform stored in the storage unit 3 as necessary. The processing of removing noise from waveform data and correcting the baseline is well known, and thus the detailed description will be omitted.

The size axis conversion unit 222 performs processing of converting the time axis into the size axis for the electrophoresis waveform subjected to preprocessing by the waveform preprocessing unit 221. At this time, the time axis is converted to the size axis using the waveform (ladder waveform) obtained from an external standard substance. The size axis may be represented, for example, in nt (nucleotide) units, or may be represented in index units.

The feature amount calculation unit 223 performs processing of calculating a feature value based on the electrophoresis waveform in which the time axis has been converted to the size axis by the size axis conversion unit 222. Although the processing of calculating this feature value will be described later, in this embodiment, the feature value corresponding to the position of a specific peak in the electrophoresis waveform is calculated.

The quality value calculation unit 224 performs processing of calculating the quality value representing the quality of RNA based on the feature value specific to the electrophoresis waveform calculated by the feature amount calculation unit 223. At this time, the feature value calculated by the feature amount calculation unit 223 may be calculated as the quality value as it is, or the quality value different from the feature value may be calculated. Further, the feature value may be converted to the quality value by performing processing, such as, e.g., linear conversion, as necessary. The quality of RNA can be evaluated based on the quality value calculated in this manner.

## 2. RNA Electrophoresis Waveform

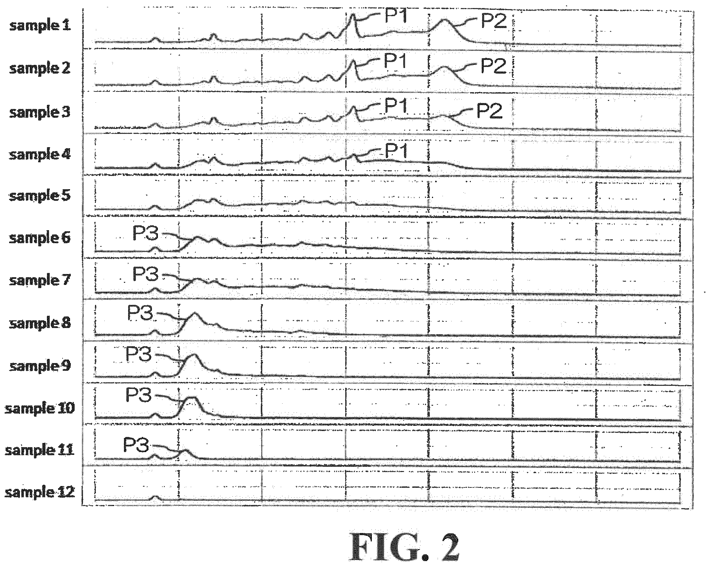

FIG. 2 is a diagram showing electrophoresis waveforms obtained using 12 (twelve) RNA different in quality as samples. The quality of sample 1 is the highest, and the quality gradually degrades toward sample 12.

It is known that the electrophoresis waveform of RNA includes the peak of 18S fragment (18S peak P1) and the peak of 28S fragment (28S peak P2). These 18S peak P1 and 28S peak P2 are characterized in that the peak intensity decreases as the RNA degrades. In the example of FIG. 2, the intensity of the 18S peak P2 in sample 1 gradually decreases in the order of samples 2, 3, 4, and so on, and is almost lost in sample 5. Further, the intensity of the 28S peak P2 in sample 1 gradually decreases in the order of samples 2, 3, 4, and so on, and is almost lost in sample 4.

On the other hand, focusing on the region on the low-molecular-weight side (the side with a shorter migration time) relative to the 18S peak in the electrophoresis waveform, in sample 6 in which the RNA was degraded to such an extent that the 18S peak P1 and the 28S peak P2 disappeared, a new peak (degradation product peak P3) generated in accordance with degradation of the RNA appears. The peak top position gradually shifts to the low-molecular-weight side while the peak intensity of this degradation product peak P3 gradually increases in the order of samples 7, 8, 9, and so on. Then, as the RNA further degrades, the peak intensity gradually decreases while the peak top position continues to gradually shift to the low-molecular-weight side in the order of samples 10, 11, and the peak intensity almost disappears in sample 12.

In this embodiment, the feature value corresponding to the position is calculated using the above-mentioned degradation product peak P3 that appears in the region (especially in the first region) on the low-molecular-weight side relative to the 18S peak in the electrophoresis waveform, and the quality of RNA is evaluated based on the calculated feature value. That is, the degradation product peak P3 appears in a region on the low-molecular-weight side relative to the 18S peak P1 in accordance with degradation of RNA after RNA has degraded to the extent that the 18S peak P1 disappears, and shifts to the low-molecular-weight side as the RNA degrades. Therefore, even for the degraded RNA, the quality can be evaluated with high accuracy on the basis of the feature value corresponding to the position of this degradation product peak P3.

## 3. First Example of Quality Evaluation

In the first embodiment of the method of evaluating the quality of RNA, the value representing the peak top position of the degradation product peak P3 is used as a feature value (peak top position feature value f1) corresponding to the position of the degradation product peak P3.

For example, when the value of the size axis is i and the intensity of the electrophoresis waveform at that size is e[i], the peak top position feature value f1 is expressed by the following formula (1). In Formula (1), R is a region (for example, first region) in which the degradation product peak P3 can occur.

$$f_1 = \arg\max_{i \in R} e[i] \quad \cdots \text{(1)}$$

That is, by calculating argmax of e[i] in the region R in which the degradation product peak P3 can occur, the value of i that maximizes e[i] in the region R is calculated as the peak top position feature value f1. The value of the peak top position feature value f1 changes (decreases) with degradation of RNA, so quality of RNA can be evaluated based on the change. Thus, the feature value (peak top position feature value f1) corresponding to the position of the degradation product peak P3 can be accurately represented using the position of the peak top of the degradation product peak P3. Therefore, based on the feature value, it is possible to accurately evaluate the quality of RNA even if it is degraded RNA.

## 4. Second Example of Quality Evaluation

In the second embodiment of the method of evaluating quality of RNA, the value showing the area ratio of each region when an area of the degradation product peak P3 is divided into a low-molecular-weight side region (FPF) and a high-molecular-weight side region (FPL) is used as the feature value (peak area ratio feature value f2) corresponding to the position of the degradation product peak P3.

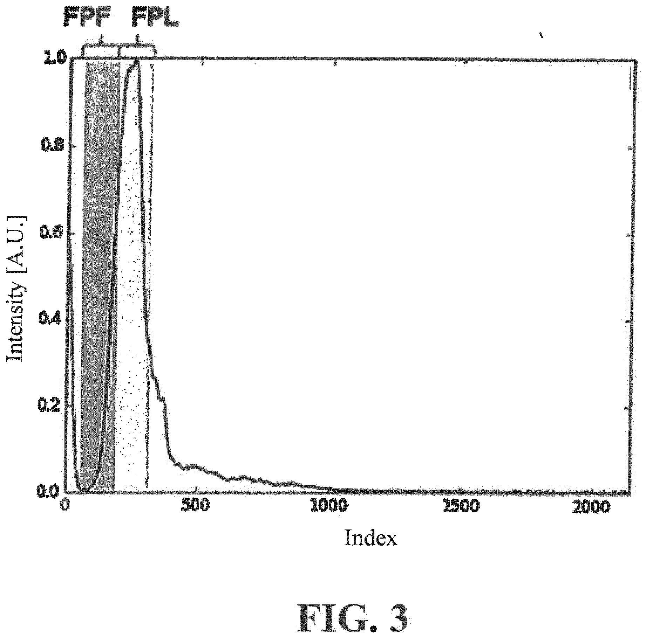

FIG. 3 is a diagram for explaining the area ratio of FPF and FPL. As shown in FIG. 3, a local region (FPF + FPL) is preset in the region R where the degradation product peak P3 can occur, and the region is bisected into FPF and FPL. In this case, the area SFPF of the degradation product peak P3 in the area of FPF is represented by the following formula (2), and the area SFPL of the degradation product peak P3 in the area of FPL is represented by the following formula (3).

$$S_{FPF} = \sum_{i \in FPF} e[i] \qquad \cdots (2)$$

$$S_{FPL} = \sum_{i \in FPL} e[i] \qquad \cdots (3)$$

Therefore, the ratio of the area SFPL of the degradation product peak P3 in the region of FPL to the area (SFPF + SFPL) of the degradation product peak P3 in the local region (FPF + FPL) is represented by the following formula (4).

$$f_2 = \frac{S_{FPL}}{S_{FPF} + S_{FPL}} \qquad \cdots (4)$$

The area ratio represented by the above formula (4) is used as the peak area ratio feature value f2. The value of this peak area ratio feature value f2 changes as the position of the degradation product peak P3 degrades due to the degradation of the RNA, so the quality of RNA can be evaluated based on the change. Thus, using the area ratio when the area of the degradation product peak P3 is divided into the low-molecular-weight side region FPF and the high-molecular-weight side region FPL, it is possible to accurately represent the feature value (peak area ratio feature value f2) corresponding to the position of the degradation product peak P3. Therefore, on the basis of the feature value, it is possible to accurately evaluate the quality of RNA even if it is a degraded RNA.

## 5. Third Example of Quality Evaluation

In the third embodiment of the method of evaluating quality of RNA, the value representing the centroid of the degradation product peak P3 is used as a feature value (peak centroid feature value f3) corresponding to the position of the degradation product peak P3. The centroid of the signal value in the region R where the degradation product peak P3 may occur is expressed by the following formula (5), and this value is calculated as a peak centroid feature value f3.

$$f_3 = \frac{\sum_{i \in R} e[i] \times i}{\sum_{i \in R} e[i]} \qquad \cdots (5)$$

The peak centroid feature value f3 represented by the above formula (5) changes as the RNA degrades and the position of the degradation product peak P3 shifts, so quality of RNA can be evaluated based on the change. As described above, Thus, by using the centroid of the degradation product peak P3, it is possible to accurately represent the feature value (peak centroid feature value f3) corresponding to the position of the degradation product peak P3. Therefore, on the basis of the feature value, it is possible to accurately evaluate the quality of RNA even if it is a degraded RNA.

## 6. Experimental Results For Each Feature Value

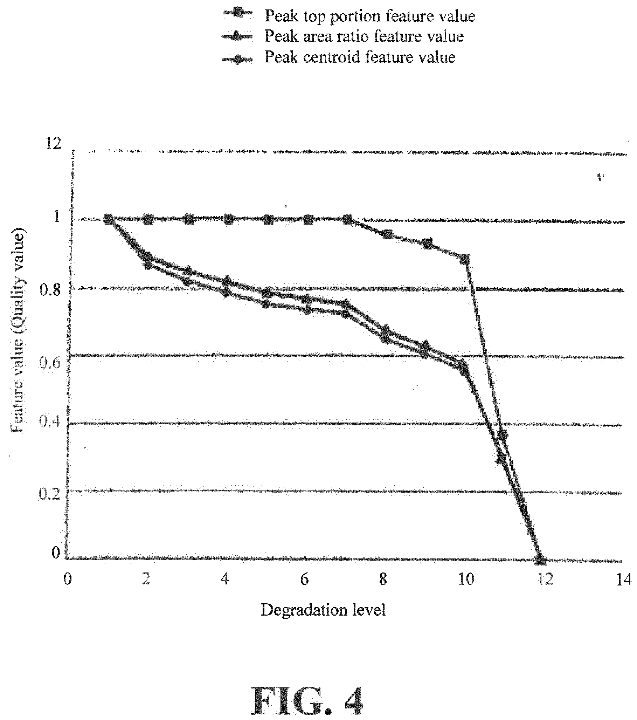

FIG. 4 shows experimental results representing the relationship between each feature value and quality of RNA corresponding to the position of the degradation product peak P3. In this experiment, the peak top position feature value f1, the peak area ratio feature value f2, and the peak centroid feature value f3 were calculated using an electrophoresis waveform of total RNA of human liver degraded in 12 steps (see FIG. 2). FIG. 4 shows the results plotting the calculated feature values (quality values) in association with each of samples 1 to 12.

As shown in FIG. 4, for any feature value, the quality of RNA decreases as it degrades. In particular, when the RNA has degraded to a degradation level of 8 or more, that is, to the extent of sample 8, the change in quality can be easily distinguished, so that it can be confirmed that the quality can be evaluated accurately even if it is degraded RNA.

## 7. Flow Chart of Quality Value Calculation Processing

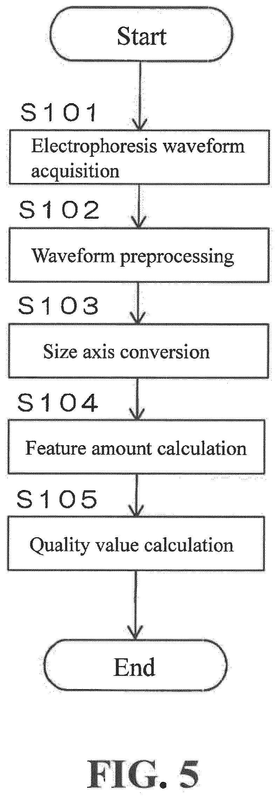

FIG. 5 is a flowchart showing processing when the data processing unit 2 calculates quality value. First, based on the detection signal from the detection unit 1, the data processing unit 2 acquires data of the electrophoresis waveform by the waveform acquisition unit 21 and stores the data in the storage unit 3 (Step S101). Thereafter, preprocessing is performed by the waveform preprocessing unit 221 on the electrophoresis waveform stored in the storage unit 3 (Step S102). Then, processing of converting the time axis into a size axis is performed by the size axis conversion unit 222 on the electrophoresis waveform subjected to preprocessing (Step S103).

In this embodiment, focusing only on the region on the low-molecular-weight side relative to the 18S peak in the electrophoresis waveform, one of the feature values, such as the peak top position feature value f1, the peak area ratio feature value f2, and the peak centroid feature value f3, is calculated by the feature amount calculation unit 223 (Step S104). Then, the feature value is converted by the quality value calculation unit 224 into a quality value representing quality of RNA (Step S105).

## 8. First Modification of Quality Value Calculation Processing

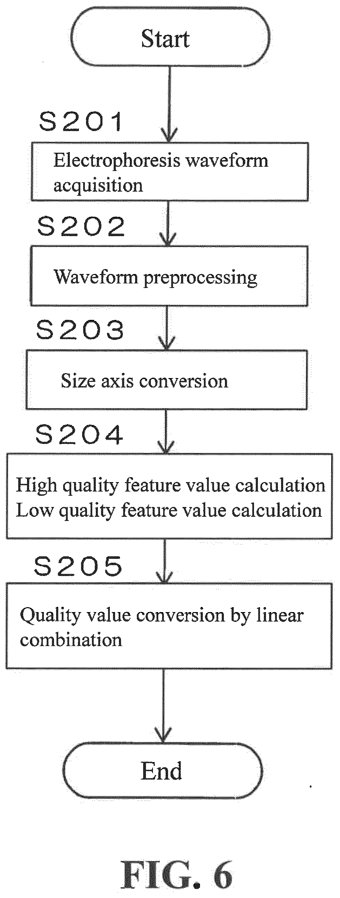

FIG. 6 is a flowchart showing processing according to a first modification when a data processing unit 2 calculates a quality value. First, based on the detection signal from the detection unit 1, the data processing unit 2 acquires data of the electrophoresis waveform by the waveform acquisition unit 21 and stores the data in the storage unit 3 (Step S201). After that, preprocessing is performed by the waveform preprocessing unit 221 on the electrophoresis waveform stored in the storage unit 3 (Step S202). Then, processing of converting the time axis into a size axis is performed by the size axis conversion unit 222 on the electrophoresis waveform subjected to preprocessing (Step S203).

In this modification, focusing on not only the region on the low-molecular-weight side of in the electrophoresis waveform relative to the 18S peak but also the 18S peak and 28S peak, the quality value representing quality of RNA is calculated. Specifically, by using not only the feature values (low quality feature values), such as, e.g., the peak top position feature value f1, the peak area ratio feature value f2, and the peak centroid feature value f3, but alto high quality feature values based on the 18S peak and the 28S peak, the quality value is calculated. In this case, the feature amount calculation unit 223 calculates, in addition to the low quality feature value, the high quality feature value by a known algorithm (Step S204).

The quality value calculation unit 224 calculates the quality value by linear combination based on the low quality feature value and the high quality feature value calculated by the feature amount calculation unit 223 (Step S205). Specifically, when the low quality feature values, such as, e.g., the peak top position feature value f1, the peak area ratio feature value f2, and the peak centroid feature value f3 are fL and the high quality feature values based on the 18S peak and 28S peak are fH, the quality value Q1 can be expressed by the following formula (6) using the coefficients C0, C1, and C2.

$$Q1 = C0 + C1fL + C2fH \quad \ldots \quad (6)$$

As described above, in this embodiment, the quality value Q1 is calculated using the feature value (low quality feature value fL) corresponding to the position of the degradation product peak P3 and the feature value (high quality feature value fH) based on the 18S peak and the 28S peak, and quality of RNA can be evaluated based on its quality value Q1. With this, until the RNA degrades to some extent, the quality of RNA can be evaluated using the high quality feature value fH, and after the RNA has degraded to some extent, the quality of RNA can be evaluated using the low quality feature value fL. Therefore, the degradation state of RNA can be evaluated over the wider range. However, the high quality feature value fH is not limited to one calculated using both the 18S peak and the 28S peak, and may be calculated using either the 18S peak or the 28S peak. In this case, the high quality feature value based on the 18S peak or the like or the high quality feature value based on the 28S peak or the like may be calculated by calculating the high quality feature value in combination with the regions other than the 18S peak and the 28S peak.

## 9. Second Modification of Quality Value Calculation Processing

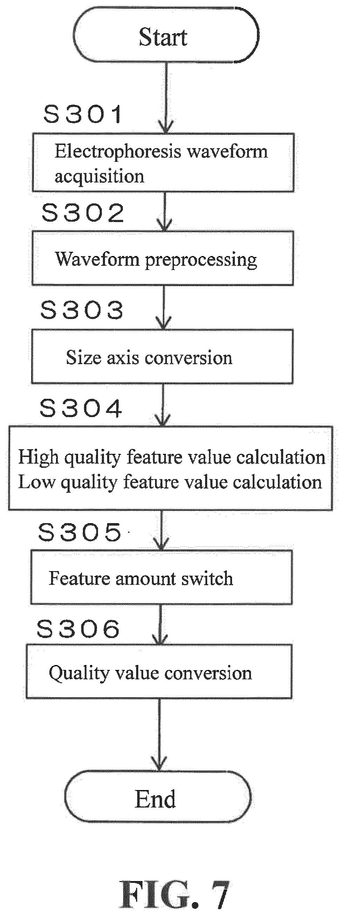

FIG. 7 is a flowchart showing processing according to a second modification when the data processing unit 2 calculates a quality value. First, based on the detection signal from the detection unit 1, the data processing unit 2 acquires data of the electrophoresis waveform by the waveform acquisition unit 21 and stores the data in the storage unit 3 (Step S301). After that, preprocessing is performed by the waveform preprocessing unit 221 on the electrophoresis waveform stored in the storage unit 3 (Step S302). Then, processing of converting the time axis into a size axis is performed by the size axis conversion unit 222 on the electrophoresis waveform subjected to preprocessing (Step S303).

In this modification, focusing on not only the region on the low-molecular-weight side relative to the 18S peak in the electrophoresis waveform but also the 18S peak and 28S peak, the quality value representing quality of RNA is calculated. Specifically, by switching the low quality RNA feature value (low quality feature value), such as, e.g., the peak top position feature value f1, the peak area ratio feature value f2, and the peak centroid feature value f3 and the high quality feature values based on the 18S peak and the 28S peak, the quality value is calculated. In this case, the feature amount calculation unit 223 calculates, in addition to the low quality feature value, the high quality feature value by a known algorithm (Step S304).

The quality value calculation unit 224 switches to either the low quality feature value or the high quality feature value according to whether or not the high quality feature value is equal to or less than a fixed value (Step S305), and the quality value is calculated based on the feature value (Step S306). Specifically, when the low quality feature values, such as, e.g., the peak top position feature value f1, the peak area ratio feature value f2, the peak centroid feature value f3 are fL and the high quality feature values based on the 18S peak and the 28 Speak are fH, the quality value Q 2 can be expressed by the following formulas (7) and (8) using the coefficients C01, C02, C1, and C2. That is, when the high quality feature value fH is larger than a constant value $\alpha$, the formula (7) is used, while when the high quality feature value fH is equal to or less than the constant value $\alpha$, the quality value Q2 is calculated using the formula (8).

$$Q2 = C01 + C1fH \quad (fH > \alpha) \qquad \ldots (7)$$

$$Q2 = C02 + C2fL \quad (fH \leqq \alpha) \qquad \ldots (8)$$

As described above, in this embodiment, the quality value Q2 is calculated using the feature value (low quality feature value fL) corresponding to the position of the degradation product peak P3 and the feature value (high quality feature value fH) based on the 18S peak and the 28S peak, and the quality of RNA can be evaluated based on its quality value Q2. With this, until the RNA degrades to some extent, the quality of RNA can be evaluated using the high quality feature value fH, and after the RNA has degraded to some extent, the quality of RNA can be evaluated using the low quality feature value fL. Therefore, the degradation state of RNA can be evaluated over the wider range. However, the high quality feature value fH is not limited to one calculated using both the 18S peak and the 28S peak, and may be calculated using either the 18S peak or the 28S peak. In this case, the high quality feature value based on the 18S peak or the like or the high quality feature value based on the 28S peak or the like may be calculated by calculating the high quality feature value in combination with the regions other than the 18S peak and the 28S peak.

In the above embodiments, the configuration has been described in which the electrophoretic analysis method according to the present invention is performed by the processing of the electrophoretic analysis device. However, the present invention is not limited to such a configuration, and at least a part of each step of the electrophoresis analysis method may be performed manually by the user.

It is also possible to provide a program (electrophoretic analysis program) for making a computer function as the above-mentioned electrophoretic analysis device. In this case, the program may be configured to be provided in a state in which it is stored in a storage medium, or the program itself may be configured to be provided.

## Description of Reference Symbols

1    detection unit
2    data processing unit
3    storage unit
21    waveform acquisition unit
22    quality evaluation processing unit
221    waveform preprocessing unit
222    size axis conversion unit
223    feature amount calculation unit
224    quality value calculation unit

## Claims

1. An electrophoretic analysis method for evaluating quality of RNA by a waveform analysis of an electrophoresis waveform, comprising:
evaluating quality of RNA on a basis of a feature value corresponding to a position of a degradation product peak (P3) that appears in accordance with degradation of RNA in a region on a low-molecular-weight side relative to an 18S peak (P1) in the electrophoresis waveform, and shifts to the low-molecular-weight side with further degradation of RNA.

2. The electrophoretic analysis method as recited in claim 1,
wherein the feature value corresponding to the position of the degradation product peak is a value representing a position of a peak top of the degradation product peak.

3. The electrophoretic analysis method as recited in claim 1,
wherein the feature value corresponding to the position of the degradation product peak is a value representing an area ratio when an area of the degradation product peak is divided into a low-molecular-weight side region and a high-molecular-weight side region.

4. The electrophoretic analysis method as recited in claim 1,
wherein the feature value corresponding to the position of the degradation product peak is a value representing a centroid of the degradation product peak.

5. The electrophoretic analysis method as recited in claim 1,
wherein the quality of RNA is evaluated using the feature value corresponding to the position of the degradation product peak and a feature value based on the 18S peak or a 28S peak.

6. An electrophoretic analysis device for evaluating quality of RNA by a waveform analysis of an electrophoresis waveform, comprising:
a quality value calculation unit (224) configured to calculate a quality value representing quality of RNA on a basis of a feature value corresponding to a position of a degradation product peak (P3) that appears in accordance with degradation of RNA in a region on a low-molecular-weight side relative to an 18S peak (P1) in the electrophoresis waveform, and shifts to the low-molecular-weight side with further degradation of RNA.

7. An electrophoretic analysis program for evaluating quality of RNA by a waveform analysis of an electrophoresis waveform, wherein, when the program is executed on a computer, the program causes said computer to execute the steps of the method according to one of claims 1 to 5.

**Patentansprüche**

1. Elektrophoretisches Analyseverfahren zur Bewertung der Qualität von RNA durch eine Wellenformanalyse einer Elektrophorese-Wellenform, umfassend:
Bewerten der Qualität der RNA basierend auf einem Merkmalswert, der einer Position eines Abbauproduktpeaks (P3) entspricht, der in Übereinstimmung mit einem Abbau der RNA in einem Bereich auf einer Seite mit niedrigem Molekulargewicht relativ zu einem 18S-Peak (P1) in der Elektrophorese-Wellenform erscheint und sich mit weiterem Abbau der RNA zur Seite mit niedrigem Molekulargewicht verschiebt.

2. Elektrophoretisches Analyseverfahren gemäß Anspruch 1,
wobei der Merkmalswert, der der Position des Abbauproduktpeaks entspricht, ein Wert ist, der eine Position einer Peakspitze des Abbauproduktpeaks darstellt.

3. Elektrophoretisches Analyseverfahren gemäß Anspruch 1,
wobei der Merkmalswert, der der Position des Abbauproduktpeaks entspricht, ein Wert ist, der ein Flächenverhältnis darstellt, wenn eine Fläche des Abbauproduktpeaks in einen Seitenbereich mit niedrigem Molekulargewicht und einen Seitenbereich mit hohem Molekulargewicht unterteilt wird.

4. Elektrophoretisches Analyseverfahren gemäß Anspruch 1,
wobei der Merkmalswert, der der Position des Abbauproduktpeaks entspricht, ein Wert ist, der einen Schwerpunkt des Abbauproduktpeaks darstellt.

5. Elektrophoretisches Analyseverfahren gemäß Anspruch 1,
wobei die Qualität der RNA unter Verwendung des Merkmalswerts, der der Position des Abbauproduktpeaks entspricht, und eines Merkmalswerts, der auf dem 18S-Peak oder einem 28S-Peak basiert, bewertet wird.

6. Elektrophoretische Analysevorrichtung zur Bewertung der Qualität von RNA durch eine Wellenformanalyse einer Elektrophorese-Wellenform, umfassend:
eine Qualitätswert-Berechnungseinheit (224), die eingerichtet ist, einen Qualitätswert, der die Qualität der RNA repräsentiert, basierend auf einem Merkmalswert zu berechnen, der einer Position eines Abbauproduktpeaks (P3) entspricht, der in Übereinstimmung mit dem Abbau von RNA in einem Bereich auf einer Seite mit niedrigem Molekulargewicht relativ zu einem 18S-Peak (P1) in der Elektrophorese-Wellenform erscheint und sich mit weiterem Abbau von RNA zur Seite mit niedrigem Molekulargewicht verschiebt.

7. Elektrophoretisches Analyseprogramm zur Bewertung der Qualität von RNA durch eine Wellenformanalyse einer Elektrophorese-Wellenform, wobei, wenn das Programm auf einem Computer ausgeführt wird, das Programm den Computer veranlasst, die Schritte des Verfahrens nach einem der Ansprüche 1 bis 5 auszuführen.

**Revendications**

1. Procédé d'analyse électrophorétique pour évaluer la qualité de l'ARN par une analyse de forme d'onde d'une forme d'onde d'électrophorèse, comprenant :
l'évaluation de la qualité de l'ARN sur la base d'une valeur de caractéristique correspondant à une position d'un pic de produit de dégradation (P3) qui apparaît en fonction de la dégradation de l'ARN dans une région d'un côté des faibles poids moléculaires par rapport à un pic 18S (P1) dans la forme d'onde d'électrophorèse, et qui se déplace vers le côté des faibles poids moléculaires avec la poursuite de la dégradation de l'ARN.

2. Procédé d'analyse électrophorétique selon la revendication 1,
dans lequel la valeur de caractéristique correspondant à la position du pic du produit de dégradation est une valeur représentant une position d'un sommet de pic du pic du produit de dégradation.

3. Procédé d'analyse électrophorétique selon la revendication 1,
dans lequel la valeur de caractéristique correspondant à la position du pic du produit de dégradation est une valeur représentant un rapport d'aire lorsqu'une aire du pic du produit de dégradation est divisée en une région de côté des faibles poids moléculaires et une région de côté des poids moléculaires élevés.

4. Procédé d'analyse électrophorétique selon la revendication 1,
dans lequel la valeur de caractéristique correspondant à la position du pic du produit de dégradation est une valeur représentant un centroïde du pic du produit de dégradation.

5. Procédé d'analyse électrophorétique selon la revendication 1,
dans lequel la qualité de l'ARN est évaluée à l'aide de la valeur de caractéristique correspondant à la position du pic du produit de dégradation et d'une valeur de caractéristique basée sur le pic 18S ou un pic 28S.

6. Dispositif d'analyse électrophorétique pour évaluer la qualité de l'ARN par une analyse de forme d'onde d'une forme d'onde d'électrophorèse, comprenant : une unité de calcul de la valeur de qualité (224) configurée pour calculer une valeur de qualité représentant la qualité de l'ARN sur la base d'une valeur de caractéristique correspondant à une position d'un pic de produit de dégradation (P3) qui apparaît en fonction de la dégradation de l'ARN dans une région sur un côté des faibles poids moléculaires par rapport à un pic 18S (P1) dans la forme d'onde d'électrophorèse, et qui se déplace vers le côté des faibles poids moléculaires avec la poursuite de la dégradation de l'ARN.

7. Programme d'analyse électrophorétique pour évaluer la qualité de l'ARN par une analyse de forme d'onde d'une forme d'onde d'électrophorèse, dans lequel, lorsque le programme est exécuté sur un ordinateur, le programme amène ledit ordinateur à exécuter les étapes du procédé selon l'une quelconque des revendications 1 à 5.

2

1

Data processing unit

21

Detection unit → Waveform acquisition unit → 

3

Electrophoresis waveform

22

Quality evaluation processing unit

221

Waveform preprocessing unit

222

Size axis conversion unit

223

Feature amount calculation unit

224

Quality value calculation unit

# FIG. 1

**FIG. 2**

FIG. 3

Peak top portion feature value
Peak area ratio feature value
Peak centroid feature value

**FIG. 4**

FIG. 5

```
                        ┌─────────────────┐
                        │      Start      │
                        └─────────────────┘
                                 │
    S201                         ▼
              ┌──────────────────────────────┐
              │ Electrophoresis waveform     │
              │ acquisition                  │
              └──────────────────────────────┘
    S202                         │
                                 ▼
              ┌──────────────────────────────┐
              │   Waveform preprocessing     │
              └──────────────────────────────┘
    S203                         │
                                 ▼
              ┌──────────────────────────────┐
              │     Size axis conversion     │
              └──────────────────────────────┘
    S204                         │
                                 ▼
         ┌────────────────────────────────────────┐
         │ High quality feature value calculation │
         │ Low quality feature value calculation  │
         └────────────────────────────────────────┘
    S205                         │
                                 ▼
         ┌────────────────────────────────────────┐
         │ Quality value conversion by linear     │
         │ combination                            │
         └────────────────────────────────────────┘
                                 │
                                 ▼
                        ┌─────────────────┐
                        │       End       │
                        └─────────────────┘
```

# FIG. 6

```
                    ┌─────────────────┐
                    │      Start      │
                    └─────────────────┘
                             │
S301                         ▼
            ┌────────────────────────────────┐
            │ Electrophoresis waveform       │
            │ acquisition                    │
            └────────────────────────────────┘
S302                         │
                             ▼
            ┌────────────────────────────────┐
            │    Waveform preprocessing       │
            └────────────────────────────────┘
S303                         │
                             ▼
            ┌────────────────────────────────┐
            │      Size axis conversion       │
            └────────────────────────────────┘
S304                         │
                             ▼
        ┌────────────────────────────────────────┐
        │ High quality feature value calculation │
        │ Low quality feature value calculation  │
        └────────────────────────────────────────┘
S305                         │
                             ▼
            ┌────────────────────────────────┐
            │     Feature amount switch       │
            └────────────────────────────────┘
S306                         │
                             ▼
            ┌────────────────────────────────┐
            │     Quality value conversion    │
            └────────────────────────────────┘
                             │
                             ▼
                    ┌─────────────────┐
                    │       End       │
                    └─────────────────┘
```

# FIG. 7

**FIG. 8**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4664280 B

- JP 5620382 B

**Non-patent literature cited in the description**

- **ANDREAS SCHROEDER**. The RIN: an RNA integrity number for assigning integrity values to RNA measurements. BioMed Central, 31 January 2006
- **VLADIMIR DENISOV**. Development and validation of RQI: an RNA quality indicator for the Experion automated electrophoresis system. Bio-Rad Laboratories, Inc., 2008

- RNA Quality Score (RQS) Calculation and Correlation to RIN. Caliper Life Sciences, Inc., 09 November 2009
- Chipping away at the chip bias: RNA degradation in microarray analysis. **AUER H. et al.** Nature Genetics. Nature Publishing Group, December 2003, vol. 35, 292-293